# EUROPÄISCHE PATENTSCHRIFT

(11) **EP 2 889 002 B1**
(45) Veröffentlichungstag und Bekanntmachung des Hinweises auf die Patenterteilung: **06.07.2016**
(21) Anmeldenummer: 14197158.0
(22) Anmeldetag: 10.12.2014
(51) Int. Cl.: A61B 6/00, A61B 6/03

(54) **Verfahren und Recheneinheit zur Messung und Darstellung der Knochendichte eines Patienten**
Method and processing unit for measuring and displaying the bone density of a patient
Procédé et unité de calcul de mesure et de représentation de la densité osseuse d'un patient

(30) Priorität: 18.12.2013 US 201314132072
(43) Veröffentlichungstag der Anmeldung: 01.07.2015
(73) Patentinhaber: Siemens Healthcare GmbH, 91052 Erlangen (DE); Siemens Medical Solutions USA, Inc., Malvern, PA 19355-1406 (US)
(72) Erfinder: Grant, Katharine Lynn Rowley, Rochester, 55901 (US); Schmidt, Bernhard, 90766 Fürth (DE)
(74) Vertreter: Maier, Daniel Oliver

(56) Entgegenhaltungen:
- EP-A1- 2 583 625
- JP-A- H04 263 842
- US-A- 5 852 647
- US-A1- 2010 135 564

## Beschreibung

Die Erfindung betrifft ein Verfahren zur Knochendichtebestimmung mit Hilfe eines CT-Systems, wobei auf Basis eines Dual-Energy-Scans eine Materialzerlegung in Knochenmaterial und mindestens eine weitere Materialkomposition ausgeführt wird, um die Knochendichte in einem vorgegebenen Bereich eines Knochens darzustellen.

Zur Bestimmung der Knochendichte eines Patienten gibt es im Stand der Technik zurzeit zwei seriöse Verfahren. Einerseits die Dual-Energie-Röntgen-Absorptiometrie (DEXA oder DXA), wie z.B. in EP-A-2583625 gezeigt, andererseits die quantitative Computertomographie (QCT), wie z.B. in US-A-2010/0135564 gezeigt.

Bei der DEXA werden von einem Patienten zwei projektive Aufnahmen mit unterschiedlichen Röntgenenergiespektren erstellt. Aufgrund der unterschiedlichen energiespezifischen Absorptionswerte für Knochenmineral und Weichteilgewebe kann der Anteil des Weichteilgewebes subtrahiert werden und die Flächenbelegung des Knochenmaterials bestimmt werden. Im Ergebnis erhält man Messwerte in g/cm² Knochenmaterial.

Unter Verwendung der beiden DEXA-Projektionsaufnahmen mit unterschiedlichen Röntgenenergiespektren kann auch eine Materialzerlegung in drei Materialkompositionen vorgenommen werden, so dass eine grobe Zusammensetzung des gesamten Körpers bestimmt werden kann. In der Regel werden dabei als Materialkompositionen Fettmasse, fettfreie Masse ohne Knochen und Knochenmasse bestimmt.

Bei der QCT werden von einem Patienten aus einer Vielzahl von Projektionsrichtungen mit um den Patienten rotierender Röntgenquelle Projektionen aufgenommen, die zwei unterschiedliche Röntgenspektren präsentieren. Mit diesen Projektionen werden zwei Volumenbilddaten unterschiedlicher Röntgenenergiespektren rekonstruiert. Mit diesen zwei Volumenbilddatensätzen kann wiederum eine Materialzerlegung ausgeführt werden, so dass am Ende eine Volumenaufnahme zur Verfügung steht, die ausschließlich das vorliegende Knochenmaterial in einer 3D-Darstellung repräsentiert. Damit ist es möglich, die tatsächliche Massendichte an Knochenmineral im Knochen zu bestimmen und die Knochendichte als spezifische Dichte in kg/cm³ anzugeben.

Problematisch ist nun, dass die in der DEXA und QCT ermittelten Werte für die Knochendichte und auch die jeweils bei diesen Untersuchungen erzeugten Aufnahmen nicht unmittelbar vergleichbar sind.

Es ist daher Aufgabe der Erfindung ein Verfahren, eine Recheneinheit und ein CT-System zu finden, mit denen durch eine QCT-Untersuchung zur DEXA-Untersuchung vergleichbare Messergebnisse erzeugt werden.

Diese Aufgabe wird durch die Merkmale der unabhängigen Patentansprüche gelöst. Vorteilhafte Weiterbildungen der Erfindung sind Gegenstand untergeordneter Ansprüche.

In der Medizin hat sich inzwischen die DEXA-Methode und die damit ermittelten Knochendichtewerte trotz ihrer größeren Fehlerquote als Goldstandard entwickelt, da eine Untersuchung nach dieser Methode kostengünstiger und weniger strahlenbelastend für den Patienten ist als eine CT-Untersuchung. Muss jedoch aus anderen medizinischen Gründen eine CT-Untersuchung durchgeführt werden, so könnte die zusätzliche Strahlenbelastung durch eine zusätzliche DEXA-Untersuchung unterbleiben, wenn durch die CT-Untersuchung zur DEXA-Methode vergleichbare Werte ermittelt werden können.

Die Erfinder haben erkannt, dass dies dadurch erreicht werden kann, dass bei einer Dual-Energy-CT-Untersuchung eine Materialzerlegung in Knochenmaterial und mindestens eine weitere Materialkomposition vorgenommen wird und auf der Basis der so erhaltenen Volumenbilddaten für die mindestens zwei Materialkompositionen die Bilderzeugung einer DEXA-Untersuchung simuliert wird. Es werden also ausgehend von den durch die CT-Untersuchung bekannten Volumendaten und den damit korrespondierenden energiespezifischen Absorptionseigenschaften zwei projektive Röntgenaufnahmen mit unterschiedlichen - für die DEXA-Methode typischen - Röntgenenergiespektren simuliert. Diese simulierten Aufnahmen können dann in den bekannten Verarbeitungsprozess der DEXA-Methode übernommen werden, so dass am Ende typische Messergebnisse der DEXA-Methode ausgegeben werden. Dem behandelnden Arzt stehen dann die routinemäßig bekannten Befundungsunterlagen zur Verfügung, die er aus der DEXA-Untersuchung kennt. Entsprechend liegt damit auch eine unmittelbare Vergleichbarkeit der Untersuchungsergebnisse vor, unabhängig davon, ob sie aus einer DEXA-Untersuchung oder einer CT-Untersuchung stammen.

Während bei der DEXA-Methode Annahmen bezüglich der volumetrischen Verteilung von Fett und Weichgewebe gemacht werden, die dann in die Auswertung der Bilddaten einfließen, können nun aufgrund der tatsächlich vorliegenden volumetrischen Bilddaten aus der Materialzerlegung der CT-Bilddaten nun tatsächlich zutreffende Korrekturfaktoren verwendet werden, um die Berechnungen nach der DEXA-Methode zu verbessern.

Gemäß einem zweiten Aspekt der Erfindung schlagen die Erfinder allerdings auch vor, den Weg über die Berechnungsmethode der DEXA-Untersuchung einzusparen und direkt aus den volumetrischen und zuvor nach Materialkompositionen verlegten CT-Bilddaten unmittelbar aus den volumetrischen CT-Bilddaten des Knochenmaterials eine Projektion, vorzugsweise in ap-Richtung, zu berechnen und hieraus Knochendichtewerte in Form einer Massenbelegung des Knochenmaterials in g/cm² zu ermitteln und auszugeben.

Demgemäß schlagen die Erfinder ein Verfahren zur Messung und Darstellung der Knochendichte eines Patienten vor, welches die folgenden Verfahrensschritte aufweist:
- Verwendung einer CT-Abtastung des Patienten mit mindestens zwei unterschiedlichen Röntgenenergiebereichen, so dass für jeden Röntgenenergiebereich separate Projektionsdaten vorliegen,
- Erzeugung von Volumendarstellungen für Knochenmaterial und mindestens eine weitere Materialkomposition,
- Berechnung zweier simulierter DEXA-Projektionsaufnahmen mit jeweils unterschiedlichem Röntgenenergiespektrum unter Verwendung der Volumendarstellungen für Knochenmaterial und die mindestens eine weitere Materialkomposition,
- Verwendung der beiden simulierten DEXA-Projektionsaufnahmen zur Bestimmung der spezifischen Knochenmassebelegung gemäß der DEXA-Methode,
- Erzeugung und Ausgabe mindestens einer Projektionsaufnahme unter Verwendung mindestens einer der simulierten DEXA-Projektionsaufnahmen und mindestens einer mittleren spezifischen Knochenmassebelegung in mindestens einem vorgegebenen Bereich der mindestens einen Projektionsaufnahme.

Vorteilhaft kann als mindestens eine weitere Materialkomposition ein Gemisch aus Fett und Weichgewebe oder alternativ einerseits Fett und andererseits Weichgewebe verwendet werden.

Weiterhin wird vorgeschlagen, bei der Bestimmung der spezifischen Knochenmassebelegung gemäß der DEXA-Methode einen Korrekturfaktor zu verwenden, der das Massenverhältnis von Fett und/oder Weichgewebe zu Knochen berücksichtigt. Besonders günstig ist es hierbei, wenn das Massenverhältnis von Fett und/oder Weichgewebe zu Knochen anhand des bei der CT-Abtastung mit Materialzerlegung gefundenen tatsächlichen Massenverhältnisses bestimmt wird.

Grundsätzlich kann zur CT-Abtastung mindestens ein Röntgenspektrum verwendet werden, das sich von jedem der zur simulierten DEXA-Methode verwendeten Röntgenspektrum unterscheidet. Unterscheiden sich die Röntgenspektren der CT-Untersuchung und die Röntgenspektren der simulierten DEXA-Untersuchung, so müssen die zur simulierten DEXA-Methode verwendeten Absorptionskoeffizienten aufgrund der Kenntnis über die verwendeten Materialkompositionen umgerechnet werden, um die auftretenden Absorption bei der simulierten DEXA-Methode zu bestimmen.

Alternativ können zur CT-Abtastung und zur simulierten DEXA-Methode identische Röntgenspektren verwendet werden, so dass die in der Materialzerlegung der CT verwendeten Absorptionskoeffizienten der Materialkompositionen unmittelbar auch für die Simulation der DEXA-Aufnahmen genutzt werden können.

Entsprechend des weiteren Aspektes der Erfindung, wonach unmittelbar die Ergebnisse der CT-Untersuchung zur Knochendichtebestimmung verwendet werden, schlagen die Erfinder auch ein Verfahren zur Messung und Darstellung der Knochendichte eines Patienten vor, welches die folgenden Verfahrensschritte aufweist:
- Verwendung einer CT-Abtastung des Patienten mit mindestens zwei unterschiedlichen Röntgenenergiebereichen, so dass für jeden Röntgenenergiebereich separate Projektionsdaten vorliegen,
- Erzeugung von Volumendarstellungen für Knochenmaterial und mindestens eine weitere Materialkomposition,
- Berechnung einer Projektion der Knochenmaterialbelegung zumindest eines Ausschnittes der Volumendarstellung der Knochendichte auf eine zweidimensionale Ebene,
- Bestimmung der durchschnittlichen Knochenmaterialbelegung in mindestens einem vorgegebenen Bereich der Projektion,
- Ausgabe der Projektion der Knochendichte und Ausgabe der Knochenmaterialbelegung in dem mindestens einen vorgegebenen Bereich der Projektion.

Es wird also hierbei nicht der Umweg über den Workflow einer DEXA-Methode mit zuvor simulierten DEXA-Aufnahmen auf der Basis in Materialkompositionen zerlegter tomographischer CT-Bilddatensätze gegangen, sondern unmittelbar die volumetrische Materialverteilung von Knochenmaterial aus einer CT-Untersuchung genutzt, um einen projektiven Bilddatensatz zu erzeugen, in dem unmittelbar die Massenbelegung von Knochenmaterial, vorzugsweise in g/cm², wiedergegeben ist. Entsprechend kann direkt aus diesem Bilddatensatz für vorgegebene Knochenbereiche eine mittlere Massenbelegung von Knochenmaterial ermittelt und angegeben werden.

Um eine möglichst ähnliche optische Darstellung zur bekannten Befundausgabe der DEXA-Methode zu erreichen, kann in der ausgegebenen Projektion in Teilbereichen ohne Knochenbelegung eine Röntgenprojektion simuliert werden, welche die projizierte Volumendarstellung der mindestens einen weiteren Materialkompensation darstellt. Somit entspricht eine derart erzeugte projektive Aufnahme, den bei der DEXA-Methode üblichen Darstellungen.

Zur Bestimmung der volumetrischen Verteilung der Materialkompositionen, insbesondere zur Erzeugung von Volumendarstellungen für Knochenmaterial und mindestens einer weiteren Materialkomposition, können bekannte Materialzerlegungsmethoden ausgeführt werden.

Der Prozess der Materialzerlegung selbst kann dabei sowohl auf Projektionsdaten als auch auf den zuvor rekonstruierten Bilddaten ausgeführt werden.

Neben dem oben geschilderten Verfahren schlagen die Erfinder auch eine Recheneinheit mit einem Speicher zur Speicherung mindestens eines Computerprogramms, das im Betrieb ausgeführt wird, vor, wobei das mindestens eine Computerprogramm das oben beschriebene Verfahren ausführen soll. Vorzugsweise wird diese Recheneinheit in unmittelbarer Verbindung mit einem CT-System verwendet.

Im Folgenden wird die Erfindung anhand der Figuren näher beschrieben, wobei nur die zum Verständnis der Erfindung notwendigen Merkmale dargestellt sind. Es werden folgende Bezugszeichen verwendet: 1: Dual-Energy-CT-System; 2: erste Röntgenröhre; 3: erster Detektor; 4: zweite Röntgenröhre; 5: zweiter Detektor; 6: Gantrygehäuse; 7: Patient; 8: Patientenliege; 9: Systemachse; 10: Recheneinheit; DE-CT_{E1}, DE-CT_{E2}: Dual-Energy-CT-Bilddaten; DEXA: DEXA-Workflow; DEXA-P_{EL}: DEXA-Projektionsdaten mit niedrigem Röntgenenergiespektrum; DEXA-P_{EH}: DEXA-Projektionsdaten mit hohem Röntgenenergiespektrum; E1, E2, EL, EH: Röntgenenergiespektren; MZ: Materialzerlegung; SIM: Simulation der DEXA-Projetion; 3D_{K}: Volumendarstellung für Knochenmaterial; 3D_{F}: Volumendarstellung für Fett, 3D_{W}: Volumendarstellung für Weichgewebe.

Es zeigen im Einzelnen:
- FIG 1:: Dual-Energy-CT-System zur Durchführung des erfindungsgemäßen Verfahrens;
- FIG 2:: Verfahrensschema eines Dual-Energy-CT-Scans mit DEXA-Auswertung;
- FIG 3:: aus CT-Bilddaten simulierte DEXA-Projektionen mit hohem und niedrigem Röntgenenergiespektrum;
- FIG 4:: Darstellung einer DEXA-Messwertausgabe auf der Basis der DEXA-Projektionen aus FIG 3.

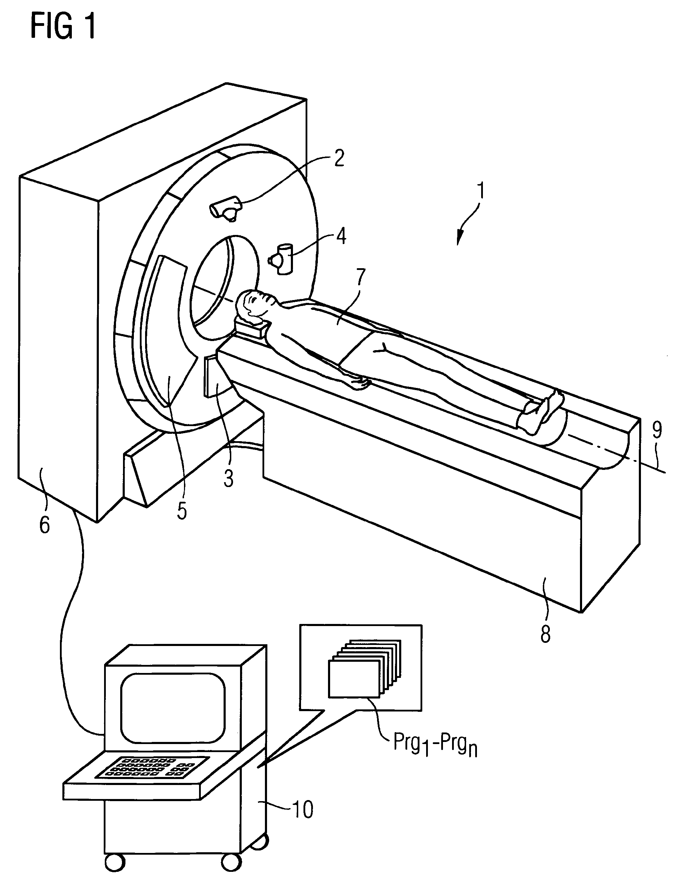

Die **Figur 1** zeigt ein schematisch dargestelltes Dual-Energy-CT-System 1 mit einem Gantrygehäuses 6, in dem sich auf einer Gantry angeordnet zwei rotierbare Strahler-Detektor-Kombinationen befinden. Die erste Strahler-Detektor-Kombination besteht aus der Röntgenröhre 2 und dem gegenüberliegenden Detektor 3. Die zweite Strahler-Detektor-Kombination befindet sich rotatorisch um 90° versetzt ebenfalls auf der Gantry und besteht aus der Röntgenröhre 4 und dem gegenüberliegenden Detektor 5. Zur Durchführung des Dual-Energy-Scans wird der Patient 7 kontinuierlich oder sequentiell mit der Drehung der Gantry mit der verschiebbaren Patientenliege 8 entlang der Systemachse 9 durch den Strahlengang der beiden Strahler-Detektor-Kombinationen 2, 3; 4, 5 geschoben und gleichzeitig mit zwei unterschiedlichen Röntgenenergiespektren abgetastet.

Aufgrund der Kenntnis des energiespezifischen Absorptionsverhaltens von Knochenmaterial und weiteren Materialkompositionen, wie Fett und Weichgewebe, kann in an sich bekannter Weise mit den so bestimmten CT-Daten eine Materialzerlegung der rekonstruierbaren tomographischen CT-Darstellungen gewonnen werden, so dass eine volumetrische Materialverteilung aus Materialkompositionen mit bekannten energiespezifischen Absorptionseigenschaften erstellt werden kann. Ausgeführt wird dies beispielsweise in der Recheneinheit 10, in der entsprechend geeignete Computerprogramme Prg₁ bis Prgₙ gespeichert sind.

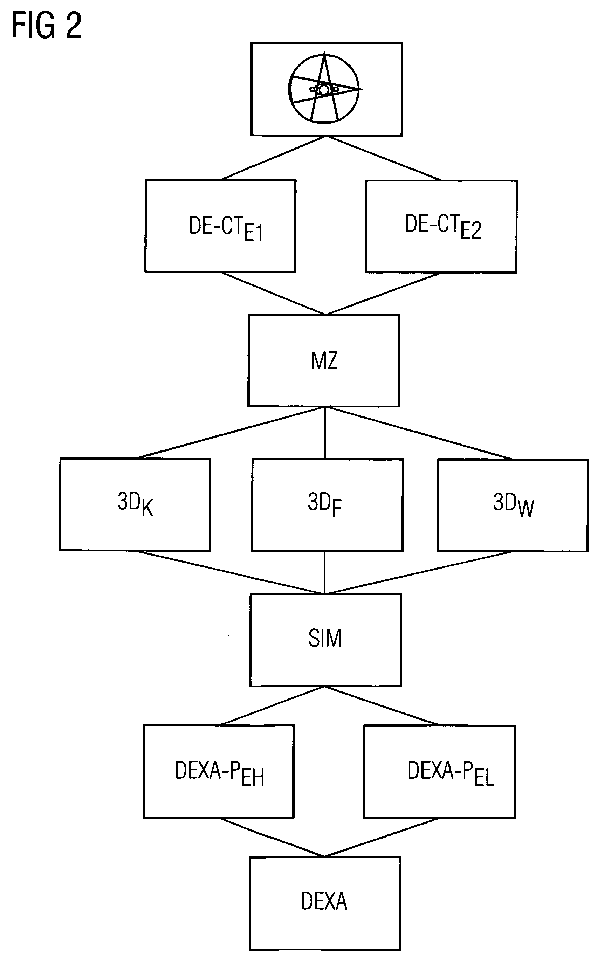

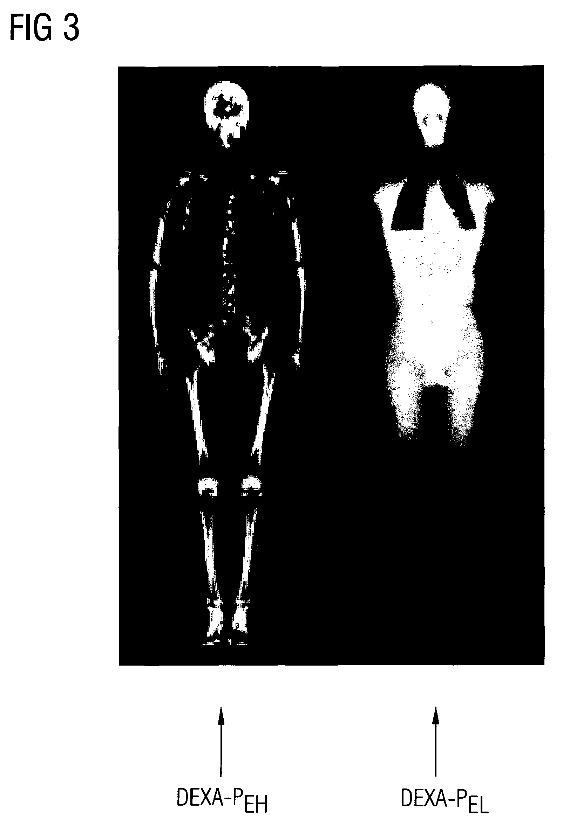

In der **Figur 2** ist ein beispielhaftes Fließschema des erfindungsgemäßen Verfahrens dargestellt. Die Dual-Energy-CT-Abtastung ist im obersten Verfahrensschritt symbolisch gezeigt. Damit ergeben sich die beiden Dual-Energy-CT-Bilddaten DE-CT_{E1} und DE-CT_{E2} aus den beiden unterschiedlichen Röntgenenergiespektren E1 und E2. Erfindungsgemäß erfolgt mit diesen CT-Bilddaten eine Materialzerlegung MZ, die als Ergebnis die dreidimensionale Materialverteilung der verwendeten Materialkompositionen, hier 3D_{K}, 3D_{F} und 3D_{W} für Knochen, Fett und Weichgewebe, ausgibt. Sind diese räumlichen Verteilungen der Materialkompositionen mit ihren energieabhängigen Absorptionskoeffizienten nun bekannt, so wird im Verfahrensschrift SIM die Röntgenaufnahme von zwei DEXA-Projektionen simuliert und es entstehen die DEXA-Projektionen DEXA-P_{EL} und DEXA-P_{EH}, wobei der Index L das niederenergetische Röntgenenergiespektrum EL und H das hochenergetische Röntgenenergiespektrum EH bezeichnen. Zur anschaulichen Darstellung sind zwei beispielhafte simulierte DEXA-Projektionen DEXA-P_{EL} und DEXA-P_{EH} in der **Figur 3** gezeigt.

Die so gewonnenen simulierten DEXA-Projektionsaufnahmen DEXA-P_{EL} und DEXA-P_{EH} können nun für den allgemein bekannten DEXA-Workflow DEXA verwendet werden, so dass dem Arzt anschließend die vertrauten Messwerte einer DEXA-Methode sowohl in der bildlichen Darstellung als auch in der Werteausgabe mit Knochendichteangaben in Form einer mittleren Massenbelegung für vordefinierte Knochenbereiche zur Verfügung stehen. Außerdem wird durch dieses Verfahren eine unmittelbare Vergleichbarkeit von originären DEXA-Befundungsdaten und Befundungsdaten einer Dual-Energy-CT-Untersuchung erreicht. In vorteilhafter Weise können ergänzend zu den DEXA-Befundungsdaten mit Massenbelegungswerten auch die korrespondierenden CT-Befundungsdaten angegeben werden, so dass der befundende Arzt ebenfalls die auf einer genaueren Mess- und Berechnungsmethode basierenden Werte zur Kontrolle zur Verfügung hat und sich gegebenenfalls auch an diese Bewertung gewöhnt.

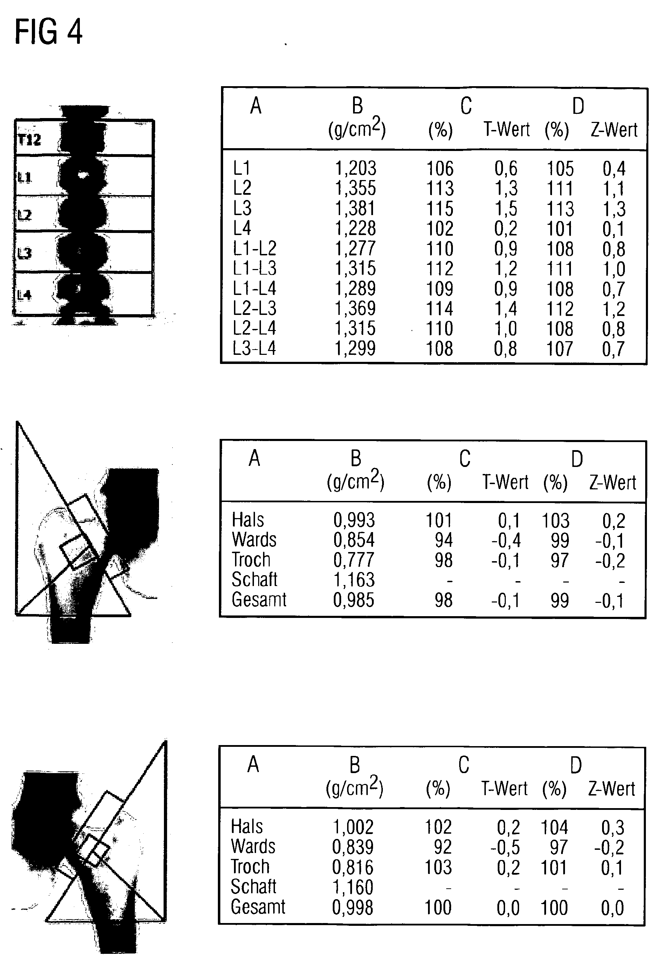

Eine beispielhafte Messwertausgabe der simulierten DEXA-Untersuchung unter Verwendung von Dual-Energy-CT-Bilddaten ist in der **Figur 4** dargestellt. Hier sind wie allgemein üblich auf der linken Seite Detailauszüge von DEXA-Projektionen einer Wirbelsäule, des rechten Femur und des linken Femur gezeigt. Rechts daneben werden in der Spalte A die untersuchten Bereiche und in der Spalte B die ermittelten mittleren Massenbelegungen der Bereiche in g/cm² angegeben. Es folgen in den Spalten unter C und D - für die Erfindung nicht relevante - Angaben zu den T-Werten und Z-Werten.

Für die Bestimmung der T-Werte gibt es verschiedenen Methoden und Geräte, die untereinander nicht vergleichbar sind. Darum wird in der Regel im Befund keine absolute Dichte oder Flächendichte angegeben, sondern es werden die Abweichungen vom Normalen in Vielfachen einer Standardabweichung, als sogenannter T-Wert (t-score) in Form einer dimensionslosen Größe angegeben. Nach der gültigen Definition der WHO liegt eine Osteoporose vor, wenn der Messwert der Knochendichtemessung mindestens 2,5 Standardabweichungen unter dem Durchschnitt der geschlechtsgleichen 30-jährigen Gesunden (peak bone mass) liegt, d.h. ein T-Wert ≤ -2,5 vorliegt. Zwischen -1 und -2,5 Standardabweichungen wird von einer Osteopenie gesprochen.

Die Angabe des T-Wertes mit Bezug auf die "peak bone mass" bringt das Problem mit sich, dass mit zunehmendem Lebensalter immer größere Bevölkerungsanteile als "krank" anzusehen wären, bei den 70-jährigen Frauen wären dies fast 50 % der untersuchten Patientinnen. Deshalb wird zusätzlich ein Wert angegeben, der sich auf gesunde Männer beziehungsweise Frauen gleichen Alters und gleicher Ethnologie bezieht, der Z-Wert. Ein normaler Z-Wert größer -1 zeigt an, dass die Knochendichte alterstypisch ist. Alter ist keine Krankheit und auch nicht behandelbar. Im Falle von sehr alten Menschen gilt auch eine erhöhte Frakturgefahr als alterstypisch. Osteologen raten deshalb bei niedrigem T-Wert aber normalem Z-Wert von medikamentösen Therapien ab und empfehlen vorbeugende Maßnahmen wie Gymnastik, Verzicht auf Sedativa, Abbau von häuslichen Stolperfallen, geeignete Sehhilfen und Gehstützen.

Insgesamt wird mit der Erfindung also vorgeschlagen, auf der Basis einer Dual-Energy-CT-Abtastung die Materialverteilung unter besonderer Beachtung des Knochenminerals im Körper eines Patienten zu bestimmen, daraus die Absorptionswerte für eine simulierte DEXA-Abtastung zu bestimmen, zwei DEXA-Projektionen mit hohem und niedrigem Röntgenenergiespektrum zu simulieren und diese zwei DEXA-Projektionen mit dem bekannten DEXA-Workflow auszuwerten, so dass im Ergebnis die üblichen Befundungsparameter einer DEXA-Untersuchung ausgegeben werden. Alternativ wird auch vorgeschlagen, auf der Basis der oben beschriebenen CT-Abtastung durch Materialzerlegung die räumliche Verteilung des Knochenminerals zu bestimmen und auf eine Ebene zu projizieren, so dass die bei der DEXA-Untersuchung typische Massenbelegung des Knochenminerals ausgegeben werden kann.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Messung und Darstellung der Knochendichte eines Patienten (7), aufweisend die folgenden Verfahrensschritte:
1.1. Verwendung einer CT-Abtastung des Patienten (7) mit mindestens zwei unterschiedlichen Röntgenenergiebereichen (E1, E2), so dass für jeden Röntgenenergiebereich (E1, E2) separate Projektionsdaten vorliegen,
1.2. Erzeugung von Volumendarstellungen für Knochenmaterial und mindestens eine weitere Materialkomposition (3D_{K}, 3D_{F}, 3D_{W}), **dadurch gekennzeichnet, dass** das Verfahren folgende Schritte aufweist:
1.3. Berechnung zweier simulierter DEXA-Projektionsaufnahmen (DEXA-P_{EH}, DEXA-P_{EL}) mit jeweils unterschiedlichem Röntgenenergiespektrum (EH, EL) unter Verwendung der Volumendarstellungen für Knochenmaterial und die mindestens eine weitere Materialkomposition (3D_{K}, 3D_{F}, 3D_{W}),
1.4. Verwendung der beiden simulierten DEXA-Projektionsaufnahmen (DEXA-P_{EH}, DEXA-P_{EL}) zur Bestimmung der spezifischen Knochenmassebelegung gemäß der DEXA-Methode,
1.5. Erzeugung und Ausgabe mindestens einer Projektionsaufnahme unter Verwendung mindestens einer der simulierten DEXA-Projektionsaufnahmen (DEXA-P_{EH}, DEXA-P_{EL}) und mindestens einer mittleren spezifischen Knochenmassebelegung in mindestens einem vorgegebenen Bereich der mindestens einen Projektionsaufnahme.

2. Verfahren gemäß dem voranstehenden Patentanspruch 1, **dadurch gekennzeichnet, dass** als mindestens eine weitere Materialkomposition ein Gemisch aus Fett und Weichgewebe verwendet wird.

3. Verfahren gemäß dem voranstehenden Patentanspruch 1, **dadurch gekennzeichnet, dass** als mindestens eine weitere Materialkomposition einerseits Fett und andererseits Weichgewebe verwendet wird.

4. Verfahren gemäß einem der voranstehenden Patentansprüche 1 bis 3, **dadurch gekennzeichnet, dass** bei der Bestimmung der spezifischen Knochenmassebelegung gemäß der DEXA-Methode ein Korrekturfaktor verwendet wird, der das Massenverhältnis von Fett und/oder Weichgewebe zu Knochen berücksichtigt.

5. Verfahren gemäß dem voranstehenden Patentanspruch 4, **dadurch gekennzeichnet, dass** der Korrekturfaktor, der das Massenverhältnis von Fett und/oder Weichgewebe zu Knochen berücksichtigt, anhand des bei der CT-Abtastung mit Materialzerlegung gefundenen tatsächlichen Massenverhältnisses bestimmt wird.

6. Verfahren gemäß einem der voranstehenden Patentansprüche 1 bis 5, **dadurch gekennzeichnet, dass** zur CT-Abtastung mindestens ein Röntgenspektrum (E1, E2) verwendet wird, das sich von jedem der zur simulierten DEXA-Methode verwendeten Röntgenspektrum (EH, EL) unterscheidet.

7. Verfahren gemäß einem der voranstehenden Patentansprüche 1 bis 5, **dadurch gekennzeichnet, dass** zur CT-Abtastung und zur simulierten DEXA-Methode die identischen Röntgenspektren (E1=EH, E2=EL) verwendet werden.

8. Verfahren zur Messung und Darstellung der Knochendichte eines Patienten (7), aufweisend die folgenden Verfahrensschritte:
8.1. Verwendung einer CT-Abtastung des Patienten (7) mit mindestens zwei unterschiedlichen Röntgenenergiebereichen (E1, E2), so dass für jeden Röntgenenergiebereich (E1, E2) separate Projektionsdaten vorliegen,
8.2. Erzeugung von Volumendarstellungen für Knochenmaterial und mindestens eine weitere Materialkomposition (3D_{K}, 3D_{F}, 3D_{W}), **dadurch gekennzeichnet, dass** das Verfahren folgende Schritte umfasst:
8.3. Berechnung einer Projektion der Knochenmaterialbelegung zumindest eines Ausschnittes der Volumendarstellung der Knochendichte (3D_{K}) auf eine zweidimensionale Ebene,
8.4. Bestimmung der durchschnittlichen Knochenmaterialbelegung in mindestens einem vorgegebenen Bereich der Projektion, 8.5. Ausgabe der Projektion der Knochendichte und Ausgabe der Knochenmaterialbelegung in dem mindestens einen vorgegebenen Bereich der Projektion.

9. Verfahren gemäß dem voranstehenden Patentanspruch 8, **dadurch gekennzeichnet, dass** in der ausgegebenen Projektion in Teilbereichen ohne Knochenbelegung ein Röntgenprojektion simuliert wird, welche die projizierte Volumendarstellung der mindestens einen weiteren Materialkompensation darstellt.

10. Verfahren gemäß einem der voranstehenden Patentansprüche 1 bis 9, **dadurch gekennzeichnet, dass** die Erzeugung von Volumendarstellungen für Knochenmaterial und mindestens einer weiteren Materialkomposition (3D_{K}, 3D_{F}, 3D_{W}) unter Verwendung eine Materialzerlegungsmethode (MZ) ausgeführt wird.

11. Verfahren gemäß dem voranstehenden Patentanspruch 10, **dadurch gekennzeichnet, dass** die Materialzerlegung (MZ) auf Projektionsdaten ausgeführt wird.

12. Verfahren gemäß dem voranstehenden Patentanspruch 10, **dadurch gekennzeichnet, dass** die Materialzerlegung auf rekonstruierten CT-Bilddaten (DE-CT_{E1}, DE-CT_{E2}) ausgeführt wird.

13. Verfahren gemäß einem der voranstehenden Patentansprüche 1 bis 12, **dadurch gekennzeichnet, dass** gleichzeitig korrespondierende Materialbelegungswerte als auch Materialdichtewerte ausgegeben werden.

14. Recheneinheit (10) aufweisend einen Speicher zur Speicherung mindestens eines Computerprogramms (Prg₁-Prgₙ), das im Betrieb ausgeführt wird, **dadurch gekennzeichnet, dass** das mindestens eine Computerprogramm (Prg₁-Prgₙ) das Verfahren gemäß einem der voranstehenden Verfahrensansprüche ausführt.

15. CT-System (1) mit einer Recheneinheit (10), aufweisend einen Speicher zur Speicherung mindestens eines Computerprogramms (Prg₁-Prgₙ), das im Betrieb ausgeführt wird, **dadurch gekennzeichnet, dass** das mindestens eine Computerprogramm (Prg₁-Prgₙ) das Verfahren gemäß einem der voranstehenden Verfahrensansprüche ausführt.

## Claims

1. Method for measuring and displaying the bone density of a patient (7), comprising the following method steps:
1.1. - Use of a CT scan of the patient (7) with at least two different x-ray energy regions (E1, E2), so that separate projection data exists for each x-ray energy region (E1, E2),
1.2. - Generation of volume displays for bone material and at least one further material composition (3D_{K}, 3D_{F}, 3D_{W}), **characterised in that** the method has the following steps:
1.3. - Calculation of two simulated DEXA projection recordings (DEXA-P_{EH}, DEXA-P_{EL}) with a different x-ray energy spectrum (EH, EL) in each instance using the volume displays for bone material and the at least one further material composition (3D_{K}, 3D_{F}, 3D_{W}),
1.4. - Use of the two simulated DEXA projection recordings (DEXA-P_{EH}, DEXA-P_{EL}) to determine the specific bone mass occupancy according to the DEXA method,
1.5. - Generation and output of at least one projection recording using at least one of the simulated DEXA projection recordings (DEXA-P_{EH}, DEXA-P_{EL}) and at least one average, specific bone mass occupancy in at least one predetermined region of the at least one projection recording.

2. Method according to the preceding claim 1, **characterised in that** a mixture of fat and soft tissue is used as at least one further material composition.

3. Method according to the preceding claim 1, **characterised in that,** on the one hand fat and on the other hand soft tissue is used as at least one further material composition.

4. Method according to one of the preceding claims 1 to 3, **characterised in that,** with the determination of the specific bone mass occupancy according to the DEXA method, a correction factor is used, which takes the mass ratio of fat and/or soft tissue relative to bones into account.

5. Method according to the preceding claim 4, **characterised in that** the correction factor, which takes the mass ratio of fat and/or soft tissue relative to bones into account, is determined with the aid of the actual mass ratio found with a material breakdown during the CT scan.

6. Method according to one of the preceding claims 1 to 5, **characterised in that** at least one x-ray spectrum (E1, E2) is used for the CT scan, which differs from each x-ray spectrum (EH, EL) used for the simulated DEXA method.

7. Method according to one of the preceding claims 1 to 5, **characterised in that** the identical x-ray spectra (E1=EH, E2=EL) are used for the CT scan and the simulated DEXA method.

8. Method for measuring and displaying the bone density of a patient (7), having the following method steps:
8.1. - Use of a CT scan of the patient (7) with at least two different x-ray energy regions (E1, E2), so that separate projection data exists for each x-ray energy region (E1, E2),
8.2. - Generation of volume displays for bone material and at least one further material composition (3D_{K}, 3D_{F}, 3D_{W}), **characterised in that** the method comprises the following steps:
8.3. - Calculation of a projection of the bone material occupancy of at least one section of the volume display of the bone density (3D_{K}) on a two-dimensional plane,
8.4. - Determination of the average bone material occupancy in at least one predetermined region of the projection,
8.5. - Output of the projection of the bone density and output of the bone material occupancy in the at least one predetermined region of the projection.

9. Method according to the preceding claim 8, **characterised in that** an x-ray projection is simulated in the output projection in sub areas without bone occupancy, which displays the projected volume display of the at least one further material compensation.

10. Method according to one of the preceding claims 1 to 9, **c h a r a c t e r i s e d in that** volume displays for bone material and at least one further material composition (3D_{K}, 3D_{F}, 3D_{W}) are generated using a material breakdown method (MZ).

11. Method according to the preceding claim 10, **c h a r a c t e r i s e d in that** the material breakdown (MZ) is executed on projection data.

12. Method according to the preceding claim 10, **characterised in that** the material breakdown is executed on reconstructed CT image data (DE-CT_{E1}, DE-CT_{E2}).

13. Method according to one of the preceding claims 1 to 12, **characterised in that** corresponding material occupancy values and also material density values are output at the same time.

14. Computing unit (10) having a memory for storing at least one computer program (Prg₁-Prgₙ), which is executed during operation, **characterised in that** the at least one computer program (Prg₁-Prgₙ) executes the method according to one of the preceding method claims.

15. CT system (1) with a computing unit (10) having a memory for storing at least one computer program (Prg₁-Prgₙ), which is executed during operation, **characterised in that** the at least one computer program (Prg₁-Prgₙ) executes the method according to one of the preceding method claims.

## Revendications

1. Procédé de mesure et de représentation de la densité osseuse d'un patient ( 7 ) ayant les stades de procédé suivant :
1.1. Utilisation d'un balayage CT du patient ( 7 ) par au moins deux plages ( E1, E2 ) d'énergie de rayons X différentes, de manière à avoir des données de projection distinctes pour chaque plage ( E1, E2 ) d'énergie de rayons X,
1.2. Production de représentations en volume de la matière osseuse et d'au moins une autre composition ( 3D_{K}, 3D_{F}, 3D_{W} ) de matière, **caractérisé en ce que** le procédé a les stades suivants :
1.3. Calcul de deux enregistrements ( DEXA-P_{EH}, DEXA-P_{EL} ) de projection DEXA simulée ayant respectivement un spectre ( EH, EL ) d'énergie de rayons X différent en utilisant les représentations en volume de la matière osseuse et de la au moins une autre composition ( 3D_{K}, 3D_{F}, 3D_{W} ) de matière,
1.4. Utilisation des deux enregistrements DEXA-P_{EH}, DEXA-P_{EL} ) de projection DEXA simulée pour déterminer une occupation spécifique de la masse osseuse suivant la méthode DEXA,
1.5. Production et émission d'au moins un enregistrement de projection en utilisant au moins l'un des enregistrements ( DEXA-P_{EH}, DEXA-P_{EL} ) de projection DEXA simulée et au moins une occupation spécifique moyenne de la masse osseuse dans au moins une partie donnée à l'avance du au moins un enregistrement de projection.

2. Procédé suivant la revendication précédente 1,
**caractérisé en ce que** l'on utilise comme au moins une autre composition de matière un mélange de graisse et de tissu mou.

3. Procédé suivant la revendication précédente 1,
**caractérisé en ce que** l'on utilise comme au moins une autre composition de matière d'une part de la graisse et d'autre part du tissu mou.

4. Procédé suivant l'une des revendications précédentes,
**caractérisé en ce que**, dans la détermination de la valuation spécifique de la masse osseuse suivant la méthode DEXA, on utilise un facteur de correction, qui tient compte du rapport massique de la graisse et/ou du tissu mou aux os.

5. Procédé suivant la revendication précédente 4,
**caractérisé en ce que** l'on détermine le facteur de correction, qui tient compte du rapport massique de la graisse et/ou du tissu mou aux os, à l'aide du rapport massique réel trouvé dans le balayage CT avec décomposition de matière.

6. Procédé suivant l'une des revendications précédentes 1 à 5, **caractérisé en ce que**, pour le balayage CT, on utilise au moins un spectre ( E1, E2 ) de rayons X qui se distingue de chaque spectre ( EH, EL ) de rayons X utilisé pour la méthode DEXA simulée.

7. Procédé suivant l'une des revendications précédentes 1 à 5, **caractérisé en ce que**, pour le balayage CT et pour la méthode DEXA simulée, on utilise les mêmes spectres ( E1=EH, E2=EL ) de rayons X.

8. Procédé de mesure et de représentation de la densité osseuse d'un patient ( 7 ) ayant les stades de procédé suivants :
8.1. utilisation d'un balayage CT du patient ( 7 ) par au moins deux plages ( E1, E2 ) d'énergie de rayons X différentes, de manière à avoir des données de projection distinctes pour chaque plage ( E1, E2 ) d'énergie de rayons X,
8.2. production de représentations en volume de la matière osseuse et d'au moins une autre composition ( 3D_{K}, 3D_{F}, 3D_{W} ) de matière, **caractérisé en ce que** le procédé comprend les stades suivants :
8.3. calcul d'une projection de l'occupation de la matière osseuse d'au moins un extrait de la représentation en volume de la densité ( 3D_{K} ) osseuse sur un plan en deux dimensions, 8.4. détermination de l'occupation moyenne de la matière osseuse dans au moins une partie donnée à l'avance de la projection,
8.5. émission de la projection de la densité osseuse et émission de l'occupation de la matière osseuse dans la au moins une partie donnée à l'avance de la projection.

9. Procédé suivant la revendication précédente 8,
**caractérisé en ce que**, dans la projection émise, on simule dans des zones partielles sans occupation d'os une projection de rayons X, qui représente la représentation en volume projetée de la au moins une autre composition de matière.

10. Procédé suivant l'une des revendications précédentes 1 à 9, **caractérisé en ce que** l'on effectue la production de représentations en volume de la matière osseuse et d'au moins une autre composition ( 3D_{K}, 3D_{F}, 3D_{W} ) de matière en utilisant un procédé ( MZ ) de décomposition de matière.

11. Procédé suivant la revendication précédente 10, **caractérisé en ce que** l'on effectue la décomposition ( MZ ) de matière sur des données de projection.

12. Procédé suivant la revendication précédente 10, **caractérisé en ce que** l'on effectue la décomposition de matière sur des données d'images CT ( DE-CT_{E1}, DE-CT_{E2} ) reconstruites.

13. Procédé suivant l'une des revendications précédentes 1 à 12, **caractérisé en ce que** l'on émet des valeurs d'occupation de matière correspondantes en même temps qu'également des valeurs de densité de matière.

14. Unité ( 10 ) informatique, ayant une mémoire de mémorisation d'au moins un programme ( Prg₁, Prgₙ ) d'ordinateur qui est exécuté en fonctionnement, **caractérisée en ce que** le au moins un programme ( Prg₁ à Prgₙ ) d'ordinateur exécute le procédé suivant l'une des revendications de procédé précédentes.

15. Système ( 1 ) CT ayant une unité ( 10 ) informatique comprenant une mémoire de mémorisation d'au moins un programme ( Prg₁ à Prgₙ ) d'ordinateur qui est exécuté en fonctionnement, **caractérisé en ce que** le au moins un programme ( Prg₁ à Prgₙ ) d'ordinateur exécute le procédé suivant l'une des revendication de procédé précédentes.
